# EUROPEAN PATENT APPLICATION

(11) **EP 2 417 967 A1**
(43) Date of publication of application: **15.02.2012**
(21) Application number: 10305843.4
(22) Date of filing: 29.07.2010
(51) Int. Cl.: A61K 9/00, A23G 4/06, A23L 1/30

(54) **A transmucosal composition containing anthocyanins for alleviating a visual discomfort**

(71) Applicant: Visiotact Pharma, 78180 Montigny-le-Bretonneux (FR)
(72) Inventor: Hadj-Slimane, Réda, 75015 Paris (FR); Lepelletier, Yves, 91200 Athis-Mons (FR); Hadj-Slimane, Tewfik, 48000 Relizane (DZ)
(74) Representative: Icosa

(57) **Abstract**

The present invention relates to a method for alleviating visual discomfort related to or caused by oxidative stress and to a composition for use in the method comprising administering via transmucosal route a composition comprising a natural extract containing anthocyanins and an agent for enhancing vigilance,, said composition being in a form adapted to transmucosal administration, said form preferably being a chewing gum, an orodispersible/orodisintegrating tablet or a buccal spray.

## Description

### FIELD OF INVENTION

The present invention relates to a method for alleviating visual discomfort related to or caused by oxidative stress and to a composition for use in the method. More specifically, the invention relates to a method for alleviating visual discomfort comprising administering via transmucosal route a composition comprising a natural extract containing anthocyanins and an agent for enhancing vigilance.

### BACKGROUND OF INVENTION

Oxidative stress corresponds to an excess of oxidant element as compared to antioxidant products, resulting in structural or formal damages, such as cellular damages. Oxidative stress is linked to an excessive production of reactive oxygen species (ROS) coupled to a deficiency in anti-radical defenses. Several scientific studies have demonstrated that oxidative stress is influenced by genetic factors (Mata et al., Invest Ophthalmol Vis Sci 2001; 42:1685-90), constitutive factors, such as aging, or environmental risks such as tobacco, UV light exposition or hypercholesterolemia (Wihlmark et al., Free radic. Biol. Med. 1997; 22:1229-34; Mares-Perlman et al., Arch Ophthalmol. 1995; 113(12):1518-23).

In retina, the oxidation of lipids linked to amino acids leads to the accumulation of lipofuscine, a fluorescent pigment. Said pigment sensitizes epithelial cells to blue light and induces the production of ROS inducing cell death by apoptosis (Wihlmark et al., Free radic. Biol. Med. 1997; 22:1229-34).

Moreover, with aging, the catalytic activity of catalase, an antioxidant enzyme participating to antioxidant protection is decreased in the retinal epithelium.

Consequently, with aging, oxidative damages in the retina result in a loss of visual acuity, a poor night vision, an enhanced light sensitivity and glare.

Non-enzymatic antioxidants exist to counterbalance the effect of oxidation. Said antioxidants are for example carotenoids (lutein, zeaxanthin...) or anthocyanins which belong to the family of flavonoids. Anthocyanins may be formed of anthocyanosides, such as for example delphinidol, cyanidol, malvidol, petunidol glucosides as well as of quinolizidinic bases.

The use of non-enzymatic antioxidant for preventing or treating oxidative stress-related eye disorders or for alleviating visual discomfort related to oxidative stress is well-known in the art. For example, anthocyanins, which can be extracted from natural vegetal species, such as for example bilberry fruits, present antioxidant properties commonly used in compositions for treating or preventing eye disorders. *In vitro,* anthocyanins protect retinal cells against oxidative stress (Dutot et al., J Fr Ophtalmol., 2008; 31(10):975-80).

WO200577176 discloses the use of anthocyanins for treating or preventing eye diseases such as Age-related Macular Degeneration (AMD), glaucoma, as well as for increasing the resistance of retinal cells to blue-light damages and for limiting lipofuscine accumulation. Anthocyanins are used to be administered through an oral way by ingestion, leading to digestive absorption of the substance. However, said absorption is not efficient, as only about 1% of total anthocyanins are systemically absorbed after oral administration of bilberry extracts containing 25% of anthocyanins.

Taking into account this issue, this invention aims at improving the bioavailability of anthocyanins present in natural, preferably vegetal, extracts. This improvement of bioavailability carries the further advantage that, according to the invention, the amount of anthocyanins to be administered to a subject for alleviation of visual discomfort is lower than that to be administered by ingestion route.

Moreover, surprisingly, the Applicant herein shows that the enhancement of the state of vigilance, for example through the administration of Vitamin C or Caffeine, in combination with anthocyanins, results in an improved alleviation of visual discomfort, especially visual discomfort related to oxidative damages.

### SUMMARY

The present invention thus relates to a method for alleviating visual discomfort related to or caused by oxidative stress and to a composition for use in the method comprising administering via transmucosal route a composition comprising a natural extract containing anthocyanins and an agent for enhancing vigilance, said composition being in a form adapted to transmucosal administration, said form preferably being a chewing gum, an orodispersible/orodisintegrating tablet or a buccal spray.

In a first embodiment of the invention, said composition for use in the method is a nutraceutical composition.

In a second embodiment of the invention, said composition for use in the method is a food or dietary supplement.

In a third embodiment of the invention, said composition for use in the method is a functional food.

In one embodiment of the invention, said agent for enhancing vigilance is caffeine.

According to an embodiment of the method of the invention, a daily amount ranging from 0.01 to 600 mg of natural extract containing anthocyanins, preferably from 0.1 to 50 mg, more preferably from 0.5 to 20 mg, may be administered to the subject. In another embodiment of the method of the invention, a daily amount ranging from 0.01 to 600 mg of natural extract containing anthocyanins, preferably from 1 to 160 mg, more preferably from 10 to 100 mg, may be administered to the subject. In one embodiment of the invention, said natural extract containing anthocyanins comprises at least 25% in weight in anthocyanins to the weight of natural extract. Anthocyanins include all anthocyanosides contained in the extract.

In a preferred embodiment of the invention, said natural extract containing anthocyanins is a vegetal extract.

In a first embodiment of the invention, said natural extract containing anthocyanins is a bilberry extract, preferably said bilberry belongs to the Ericaceae family, gender *Vaccinium,* more preferably the bilberry is *Vaccinium myrtillus.*

In a second embodiment of the invention, said natural extract is a gooseberry extract, preferably said gooseberry belongs to the Grossulariaceae family, gender *Ribes.*

In one embodiment of the invention, said composition is such that a daily amount ranging from 0. 1 to 200 mg of caffeine, preferably from 1 to 150 mg, more preferably from 10 to 100 mg, may be administered to the subject, with combination to anthocyanins. In a particular embodiment of the invention, caffeine is synthetic caffeine, or is from a natural extract of guarana.

In one embodiment of the invention, the composition further comprises one or more of the following ingredients:
- Vitamin C, in an amount ranging from 0.1 to 1000 mg, preferably from 1 to 300 mg, more preferably from 10 to 280 mg, even more preferably about 240 mg; according to another embodiment, the amount of Vitamin C ranges from 0.1 to 240 mg,
- lutein and zeaxanthin in a 5/1 ratio, in an amount ranging from 0.01 to 12 mg, preferably from 0.05 to 6 mg, more preferably from 0.1 to 1.2 mg,
- taurin, in an amount ranging from 0.1 to 200 mg, preferably from 1 to 100 mg, more preferably from 10 to 50 mg,
the indicated amounts being daily amounts to be administered.

In a particular embodiment of the invention, the source of Vitamin C is ascorbic acid, sodium ascorbate or a mix thereof.

In a particular embodiment of the invention, lutein and/or zeaxanthin are partially or totally substituted with meso-zeaxanthin, astaxanthin or a mix thereof.

In one embodiment of the invention, visual discomfort to be alleviated is related to oxidative stress, said oxidative stress being due for example to photo-oxidation or photoreceptor overstimulation, especially of the retina.

In a particular embodiment of the invention, said visual discomfort is poor night vision, enhanced light sensitivity or glare.

### DEFINITIONS

In the present invention, the following terms have the following meanings:
- "Nutraceutical": a product isolated or purified from comestibles. A nutraceutical is demonstrated to have a physiological benefit or provide protection against physiological disorder or discomfort;
- "Food or Dietary supplement": a product that contains a vitamin, mineral, herb or other botanical, amino acid, concentrate, metabolite, constituent, extract, or combinations of these ingredients;
- "Functional food": any modified food or food ingredient that may provide a benefit or provide protection against physiological disorder or discomfort; beyond the traditional nutrients it contains;
- "Transmucosal administration": the administration of a product through or across a mucosal tissue. In the meaning of this invention preferred mucosal tissues are buccal, sublingual, gingival and palatal tissues. According to the invention, very preferred transmucosal administration is buccal administration. In this case, the administered substance reaches the systemic circulation through or across the mucosal tissues of the buccal cavity but is not ingested nor swallowed;
- "Oral administration": the administration of a product in the mouth cavity followed by the swallowing of the product. In this case, the substance reaches the systemic circulation through a digestive absorption;
- "Orodispersible/orodisintegrating tablet": A tablet for oral administration, whose oral administration results in its dispersion or disintegration within the mouth;
- "Chewing gum": a type of gum to be chewed formed in many different shapes and sizes (balls, sticks...) and made of a base comprising a natural latex product or synthetic rubber, to which are incorporated food colourings, flavourings, preservatives and other additives;
- "Oxidation": a reaction in which the atoms in an element lose electrons and the valence of the element is correspondingly increased. In a biological context, oxidation is usually linked to the reaction of a substance with oxygen, leading to the production of reactive oxygen species;
- "photo-oxidation": phenomenon of oxidation facilitated or provoked by light (for example sunlight);
- "Oxidative stress": imbalance between the production of reactive oxygen species in one hand and in the other hand the detoxification of the reactive intermediates and/or the repair of the resulting damages;
- "Oxidant": an agent that oxidizes another agent;
- "Antioxidant": an agent that diminishes or avoids the oxidation of other substances. An antioxidant may protect cells against the effect of reactive oxygen species;
- "Photo-receptor overstimulation": an excessive stimulation of the photoreceptor, which could lead to physiological problems;

### DETAILED DESCRIPTION

The present invention relates to a method for alleviating visual discomfort related to or caused by oxidative stress and to a composition for use in the method. In one embodiment of the invention, said visual discomfort related to or caused by oxidative stress is due to photo-oxidation or photoreceptor overstimulation, especially of the retina. In another embodiment of the invention, said visual discomfort related to oxidative stress is selected in a group comprising, but not limited to, a poor night vision, an enhanced light sensitivity and glare.

The present invention also relates to a composition comprising a natural extract containing anthocyanins and an agent for enhancing vigilance, said composition being in a form adapted to transmucosal administration, preferably to buccal administration, said form preferably being a chewing gum, an orodispersible/orodisintegrating tablet or a buccal spray. The transmucosal administration allows an enhanced and prolonged absorption of anthocyanins and thus improves the bioavailability of anthocyanins. The present invention also relates to the use of said composition for alleviating a visual discomfort related to or caused by oxidative stress in a subject in need thereof.

In a first embodiment of the invention, said composition for use in the method is a nutraceutical composition.

In a second embodiment of the invention, said composition for use in the method is a food or dietary supplement.

In a third embodiment of the invention, said composition for use in the method is a functional food.

The present invention also relates to a composition consisting of a natural extract containing anthocyanins and an agent for enhancing vigilance for use in alleviating visual discomfort, said composition being in a form adapted to transmucosal administration, said form preferably being a chewing gum, an orodispersible/orodisintegrating tablet or a buccal spray. Said composition consisting of a natural extract containing anthocyanins and an agent for enhancing vigilance is a nutraceutical composition, a food or dietary supplement, or a functional food.

In one embodiment of the invention, said agent for enhancing vigilance is at least one of caffeine, taurin and Vitamine C.

In one embodiment of the invention, said agent for enhancing vigilance is caffeine. Caffeine acts as a stimulating agent of the central nervous system and of cardiovascular system. Caffeine is used for reducing physical fatigue, for enhancing attention in order to avoid slumber and for insuring a good corporal coordination.

In a preferred embodiment, the composition of the invention includes caffeine, either from a natural extract such as for example guarana or not from a natural extract (i.e. synthetic caffeine), or both.

According to the invention, the composition is such that a daily amount ranging from 0.1 to 200 mg of caffeine, preferably from 1 to 150 mg, more preferably from 10 to 100 mg may be administered to the subject.

Anthocyanins are flavonoids with antioxidant properties. *In vitro,* anthocyanins protect retinal cells from oxidative stress. *In vivo,* anthocyanins contribute to the regeneration of rhodopsin, and thus help to maintain the visual acuity and to protect the eye against photo-oxidation. Anthocyanins have a beneficial effect on vision, and contribute to the protection or repair of diabetic retinopathies and glaucoma (Dutot et al., J Fr Ophtalmol 2008; 31(10):975-80).

According to the invention, the composition is such that a daily amount ranging from 0.01 to 600 mg of natural extract containing anthocyanins, preferably from 0.1 to 50 mg, more preferably from 0.5 to 20 mg may be administered to the subject. In another embodiment, the composition is such that a daily amount ranging from 0.01 to 600 mg of natural extract containing anthocyanins, preferably from 1 to 160 mg, more preferably from 10 to 100 mg may be administered to the subject. In one embodiment of the invention, said natural extract is standardized to at least 25% in weight in anthocyanins titrated on delphinidol.

In a preferred embodiment of the invention, said natural extract containing anthocyanins is a vegetal extract.

In one embodiment of the invention, said natural extract containing anthocyanins is selected from the group comprising, but not limited to, *Vaccinium Myrtillus* (bilberries i.e. "bog whortleberries"), *Rubus spp* (blackberry i.e. "boysenberries" from north America), *Rubus occidentalis* (black raspberry from north America), *Vaccinium corymbosum* (blueberry) , *Vaccinium macrocarpon* (American cranberry), *Vaccinium oxycoccus* (European cranberry), *Rubus idaeus* (European cranberry), *Fragaria X ananassa* (European cranberry), *Prunus virginiana* (berry i.e. "chokecherry" from North American tribal communities), *Viburnum trilobum* (berry i.e. "highbush cranberry " from North American tribal communities), *Amelanchier alnifolia* (berry i.e. "serviceberry " from North American tribal communities), *Shepherdia argentea* (berry i.e. "silver buffaloberry " from North American tribal communities), *Rubus articus* (arctic bramble), *Ribes nigrum* (black currant), *Rubus chamaemorus* (cloudberries), *Empetrum nigrum, Empetrum hermaphroditum* (crowberries), *Sambucus spp.* (elderberries), *Ribes uva-crispa* (gooseberry), *Vaccinium vitis-idaea* (lingonberries), *Rubus loganobaccus* (loganberry), *Sorbus spp* (Rowan berries), *Hippophae rhamnoides* (sea buckthorn), *Punica granatum* (pomegranate), *Lycium barbarum* (goji berries), *Garcinia mangostana* (mangosteen), *Euterpe oleraceae* (Brazilian açaí berry), *Aristotelia chilensis* (Chilean maqui berry).

In a preferred embodiment of the invention, said natural extract is a gooseberry extract, preferably said gooseberry belongs to the Grossulariaceae family, gender *Ribes.*

In a more preferred embodiment of the invention, said natural extract containing anthocyanins is a bilberry extract. Even more preferably, said bilberry belongs to the

Ericaceae family, gender *Vaccinium.* Even more preferably, said bilberry is *Vaccinium myrtillus.*

Bilberry extracts usually comprise about 25% (w/w) in anthocyanins and are commercially available. In a preferred embodiment, bilberry extract is from NATUREX ("Extrait de myrtille 25%", Naturex SA, Avignon, France).

In one embodiment of the invention, the composition of the invention further comprises Vitamin C. Vitamin C is a non-enzymatic water-soluble antioxidant. Vitamin C may act as an antioxidant agent in the eye. Vitamin C may also help to counteract the risk of deterioration of the visual functions (AREDS reports n°8, Arch Ophtalmol, 2001; 119:1417-36).

In an embodiment, the composition is such that a daily amount ranging from 0.1 to 1000 mg of Vitamin C, preferably from 1 to 300 mg, more preferably from 10 to 280 mg, even more preferably about 240 mg may be administered to the subject. In another embodiment, the composition is such that a daily amount ranging from 0.1 to 240 mg of Vitamin C may be administered to the subject.

According to the present invention, ascorbic acid, sodium ascorbate or a mix thereof can be present in the composition of the invention, as a source of vitamin C. Preferably, ascorbic acid is used as the source of vitamin C.

In one embodiment, the composition of the invention further comprises lutein and zeaxanthin. Lutein and zeaxanthin are macular pigments representing 99% of total pigments of the macula, which belong to the family of carotenoids. Lutein and zeaxanthin may exhibit on one hand an indirect antioxidant effect through their capacity to absorb blue light particularly aggressive for photoreceptor and on the other hand direct antioxidant properties. Scientific studies have shown that lutein consummation may improve eye-sensitivity to contrasts, and decreases glares.

In one embodiment, the composition is such that a daily amount ranging from 0.01 to 12 mg of lutein and zeaxanthin, preferably from 0.05 to 6 mg, more preferably from 0.1 to 1.2 mg may be administered to the subject.

In a preferred embodiment, lutein and zeaxanthin are present in a 5/1 ratio.

Purified lutein is commercially available (FloraGlo® from FluraGloLutein).

In an embodiment of the invention, lutein is partially or totally substituted by meso-zeaxanthin, astaxanthin or a mix thereof.

In an embodiment of the invention, zeaxanthin is partially or totally substituted by meso-zeaxanthin, astaxanthin or a mix thereof.

Meso-zeaxanthin is a stereo-isomer of zeaxanthin, synthesized in the eye from lutein. It might be more efficient than lutein for essential activities in the center of the macula.

Astaxanthin is a carotenoid antioxidant with photo-protective activities. Its antioxidant properties may be more efficient than those of zeaxanthin or beta-carotene. Scientific results demonstrated that astaxanthin could be useful for preventing and treating neuronal lesions in AMD (US5.527.533).

In one embodiment, the composition of the invention further comprises taurin.

Taurin (aminoethanesulfonic acid) plays a major role in development and survey of retinal neurons. Taurin may also protect retinal cells in case of diabetic retinopathies or photo-induced stress through antioxidant properties (Yu et al., Neurochem Res 2008; 33(3):500-7; Yu et al., Br J Nutr 2007; 98(4):711-9). Deficit in taurin causes deprotection of retina from photo-oxidation resulting in loss of visual acuity.

In one embodiment of the invention, the composition is such that a daily amount ranging from 0.1 to 200 mg of taurin, preferably from 1 to 100 mg, more preferably from 10 to 50 mg may be administered to the subject.

In a preferred embodiment, highly purified taurin (minimum 98.5%) is used.

The present invention also relates to a chewing gum or to an orodispersible/orodisintegrating tablet. Due to the size of such products, the quantity of active components is limited to 50 mg per gum or per tablet. The recommended daily consummation ranges from 10 to 20 chewing gums or from 1 to 2 orodispersible/orodisintegrating tablets. The daily consummation is limited to the maximum daily amount for each ingredient.

### BRIEF DESCRIPTION OF THE DRAWINGS

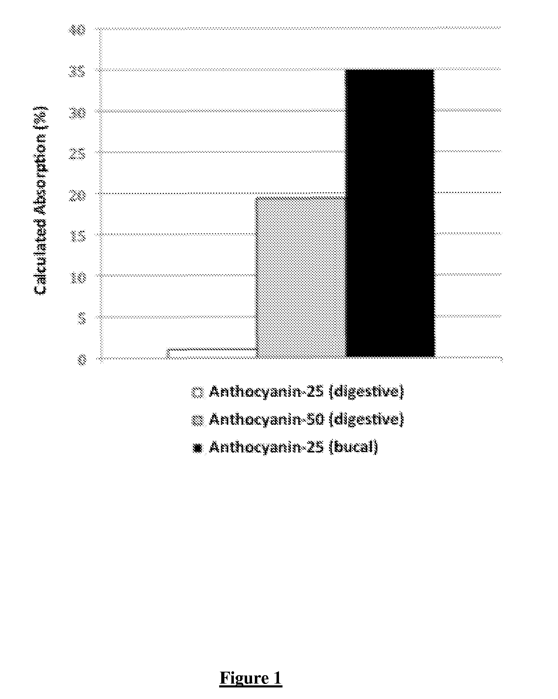

Figure 1 is a graph showing comparative data of anthocyanin absorption, with regard to the administration mode and anthocyanin amount.

### EXAMPLES

The present invention is further illustrated by the following examples.

### Example 1: Composition of the invention.

The bilberry extract used in the following examples of composition are from Naturex (Naturex SA, Avignon, France) and contained 25% in weight of anthocyanins to the total weight of the extract.

FloraGlo^{®} is from FloraGloLutein (KEMIN Health, 600E, Court Ave, Des Moines, USA) and contained Lutein/Zeaxanthin in a 5/1 ratio.

| | Composition number | | | | | | | | | | |
|---|---|---|---|---|---|---|---|---|---|---|---|
| Nutriment | 1 | 2 | 3 | 4 | 5 | 6 | 7 | 8 | 9 | 10 | 11 |
| Bilberry extract | 12.5 | 12.5 | 12.5 | 12.5 | 12.5 | 12.5 | 12.5 | 12.5 | 12.5 | 12.5 | 12.5 |
| Caffeine | 100 | 0 | 0 | 0 | 100 | 0 | 0 | 100 | 0 | 100 | 100 |
| Vitamin C | 0 | 240 | 0 | 0 | 0 | 240 | 240 | 240 | 240 | 240 | 240 |
| Taurin | 0 | 0 | 0 | 10 | 0 | 0 | 10 | 0 | 10 | 0 | 10 |
| Lutein/Zeaxanthin | 1.2 | 0 | 1.2 | 0 | 0 | 1.2 | 0 | 0 | 1.2 | 1.2 | 1.2 |

The amounts are indicated in mg per daily administration.

### Example 2: Chewing gum

| Ingredients | Function | Weight (mg) |
|---|---|---|
| Xylitol | Sweetener | 367,20 |
| Sorbitol | Sweetener | 356,40 |
| Gum base | gum | 285,88 |
| Mannitol | Sweetener | 72 |
| Sirup of maltitol | Sweetener | 68 |
| Arum menthol | Aroma | 33,75 |
| Arum mint | Aroma | 21 |
| Arabic gum | Thickening agent | 12 |
| lecithine | Emulsifier | 8,50 |
| Dioxide titanium | colouring agent | 8 |
| Acesulfam K | Sweetener | 2,55 |
| Mint | Aroma | 2,50 |
| Aspartame | Sweetener | 0,85 |
| Bilberry extract | Active ingredient | 1,25 |
| Caffeine | Active ingredient | 10 |
| Lutein | Active ingredient | 0,1 |
| Zeaxanthin | Active ingredient | 0,02 |
| TOTAL | | 1250 |

### Example 3: Orodispersible/orodisintegrating tablet

- 12.5 mg of Bilberry extract
- 100 mg of caffeine
- 1 mg of lutein
- 0.2 mg of zeaxanthin

### Example 4: Biological examples

### Material

Rats OFA, Sprague-Dawlay
The stick gum administered to Rats contains 5 mg of vegetal extract titrated at 25% of anthocyanosides (i.e. 1.250 µg of anthocyanosides).
Trypsin and collagenase are from Worthington Biochemical Corporation, Lakewood, New Jersey

### Method

### Diets.

The Sprague Dawley rat (10 Males) used extensively in medical research weighing 450-520g were received and placed separately in cage in room temperature controlled (22°C). This rat species is chose for its calmness and its easy handling. Rats were maintained in dark periode condition and fed a diet (755 g/kg wheat starch, 150 g/kg casein, 50 g/kg peanut oil, 35 g/kg AIN-93M mineral mixture, 10 g/kg AIN-76A vitamin mixture) during 2 weeks (Felgines, C. et al. J. Nutr 2002).

### Anthocyanin stability and detection.

As anthocyanins are particularly susceptible to pH variations and are unstable at intestinal pH, collected samples were analyzed by HPLC after acidification with 240 mmol/L HCl, to allow the comparison with intestinal administration. Plasmatic anthocyanins levels were assessed after plasma separation of blood samples by centrifugation and extracted with a solid-phase extraction cartridge (Sep-Pak C18 Plus) and then analyzed by HPLC possessing a photodiode array detector and an UV-visible detector (785A, Perkin-Elmer) at 524 nm. Spectrum was compared to Cyanidin 3-glucoside that represent 98% of total anthocyanins. Anthocyanin metabolite analysis was carried out by HPLC-electrospray ionization (ESI)-MS-MS analysis and performed on a HPLC system equipped with MS-MS detection (API 2000). Anthocyanin metabolites were detected according to the respective m/z values of their parent and product ions.

Absorption from mucosal epithelium and serum was estimated by calculating the difference between anthocyanin quantity contained in bucal gum patch administered and the amount recovered at the end in samples.

### Buccal administration process

Rats (OFA, Sprague-Dawlay) received a lingual administration of anthocyanins with an orodispersible gum containing 1,25 mg of anthocyanin. The stick gum was deposited at the buccal mucosa area.

Peripheral blood was collected 1 hour post-administration, centrifuged and plasma was acidified through formic acid addition. After acidification, plasma samples were conserved at -80°C and analyzed by LC-MS/MS (Liquid Chromatography - Mass Spectrometer/ Mass Spectrometer).

In parallel, epithelium and conjunctive tissues were collected 45 minutes post-administration, placed in formic acid and eventually conserved at -80°C. Before LC-MS/MS analyze, tissues were washed in PBS (phosphate buffered saline) and homogenized on ice. Protein concentrations were determined by Lowry method. To facilitate tissues preparation, samples were homogenized and treated with 2.5% trypsin and 2% collagenase during 1.5h at 37°C, and then acidified with formic acid.

Treatment with formic acid does not affect anthocyanins stability.

Absorption was estimated by calculating the administering/recovering anthocyanosides estimated concentration in samples ratio. Quantification was adjusted in respect of residual anthocyanoside quantity contained in buccal washing (anthocyanosides extracted from stick gum not absorbed and residual non dissolved stick gum).

### Digestive administration process

Digestive administration of anthocyanosides is done as previously described (S Talavera, C Felgines, O Texier et al. J. Nutr. 134: 2275-2279, 2004).

Briefly, samples were collected at the beginning and after perfusion period (45 min), and anthocyanins were analyzed by HPLC as previously described. Intestinal perfusion method (Crespy, V., Morand, C., Manach, C., et al. Am. J. Physiol. 277: G120 -G126, 1999) is done using bile duct cannulation consist in jejunal plus intestine ileal segment (flexura duodenojejunalis to the valvula ileocecalis) perfusion. This segment was perfused for 45 min at a flow rate of 0.75 mL/min with a physiological buffer containing KH₂PO₄ (5 mmol/L), K₂HPO₄ (2.5 mmol/L), NaHCO₃ (5 mmol/L), NaCl (50 mmol/L), KCl (40 mmol/L), CaCl₂ (2 mmol/L), MgSO₄ (1 mmol/L), tri-potassium citrate (10 mmol/L), glucose (12 mmol/L), glutamine (2 mmol/L), and taurocholic acid (1 mmol/L), pH 6.6, at 37°C supplemented with blackberry extract containing 25% and 50%, respectively. Absorption was estimated by calculating the administrating/recovering anthocyanins ratio.

Extract containing 25% of Anthocyanins is purchased from Naturex SA (Avignon, France). Extract containing 50% of Anthocyanins is purchased from Ferlux Mediolanum SA (Cournon d'Auvergne, France).

Anthocyanosides concentration is based on Delphinidine (C₁₅H₁₁ClO₇, PM = 338,70g.mol⁻¹).

### References

Felgines, C., et al. (2002). Blackberry anthocyanins are slightly bioavailable in rats. J. Nutr. 132: 1249 -1253
Talavera S., et al. (2004). Anthocyanins are efficiently absorbed from the small intestine in rats. J. Nutr. 134: 2275-2279
Crespy, V., et al. (1999). Part of quercetin absorbed in the small intestine is conjugated and further secreted in the intestinal lumen. Am. J. Physiol. 277: G120 -G126

### Results

### Buccal absorption:

LC-MS/MS results, corresponding to the absorption of anthocyanin are presented on tables 1 and 2. Table 1 presents the amount of anthocyanin in the tissues, and table 2 presents the amount of anthocyanin in the plasma.

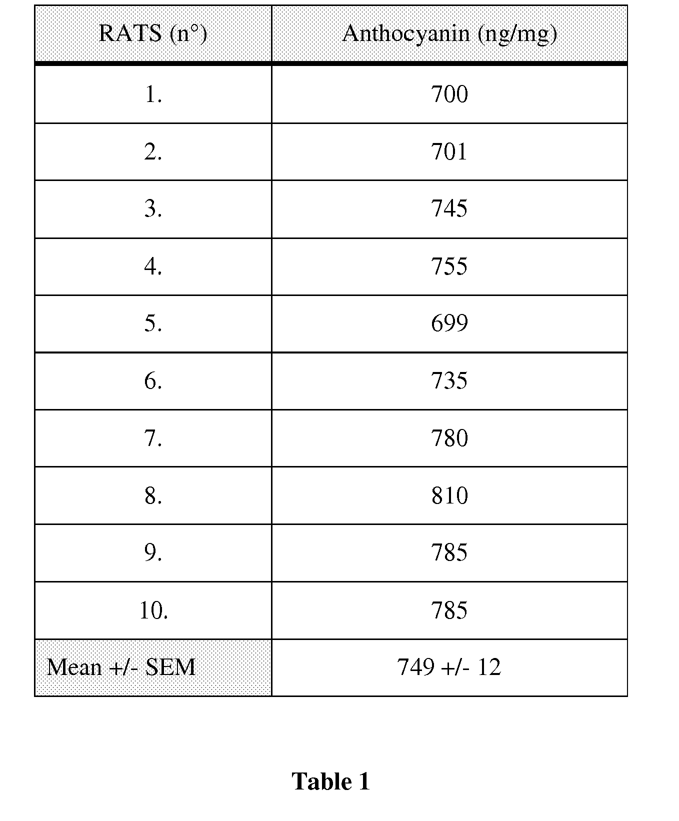

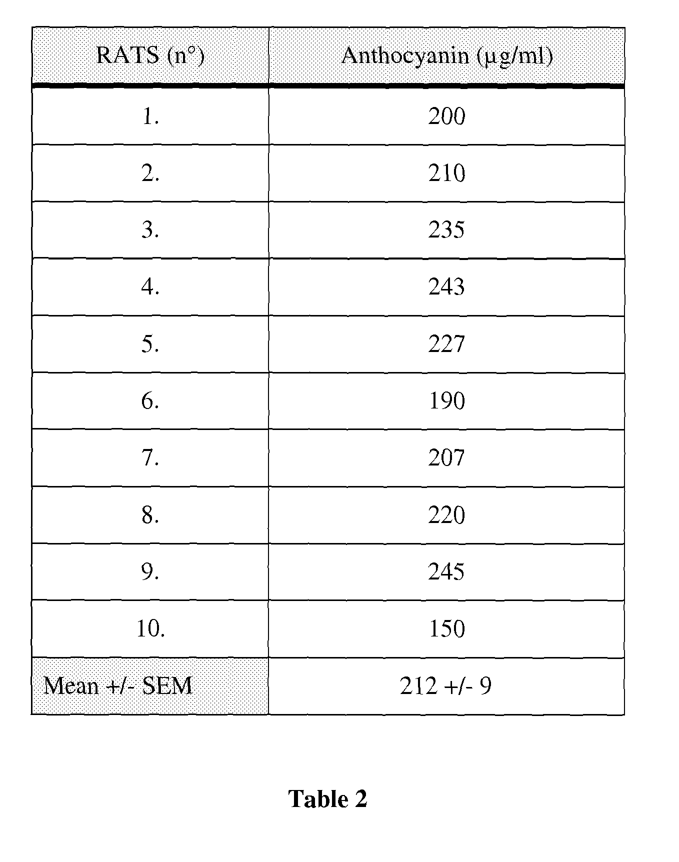

### Digestive absorption:

LC-MS/MS results, corresponding to the absorption of anthocyanin are presented on tables 3 and 4. Table 3 presents the amount of anthocyanin in the tissues, ant table 4 presents the amount of anthocyanin in the plasma.

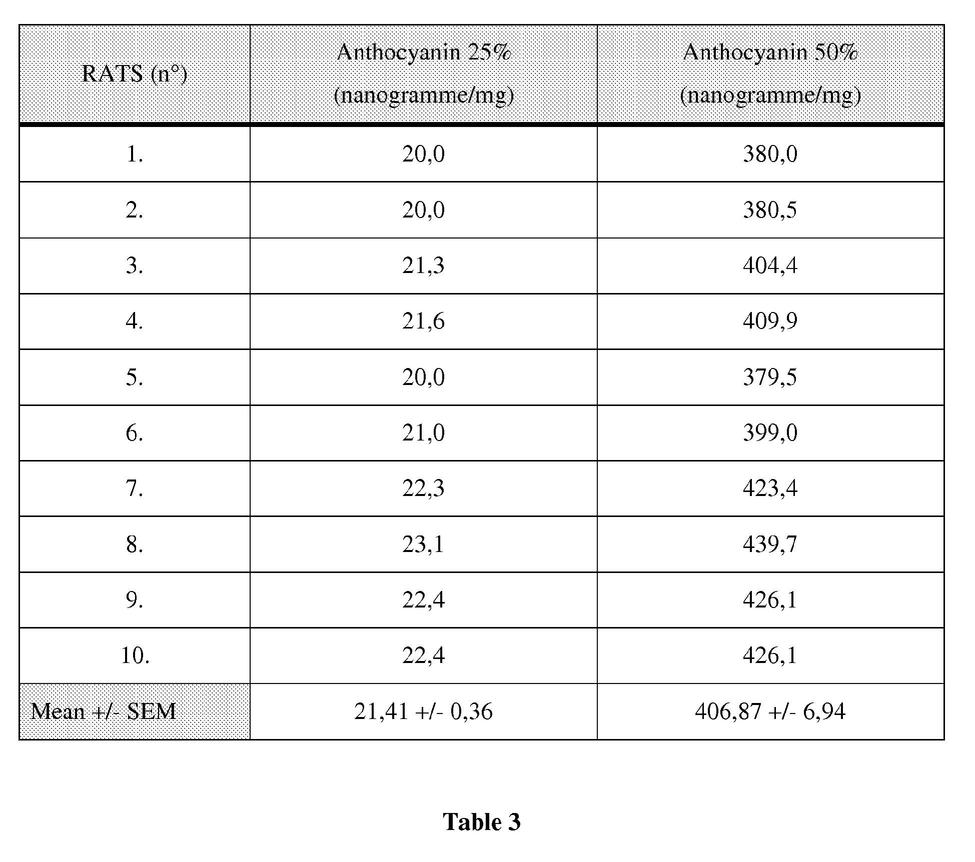

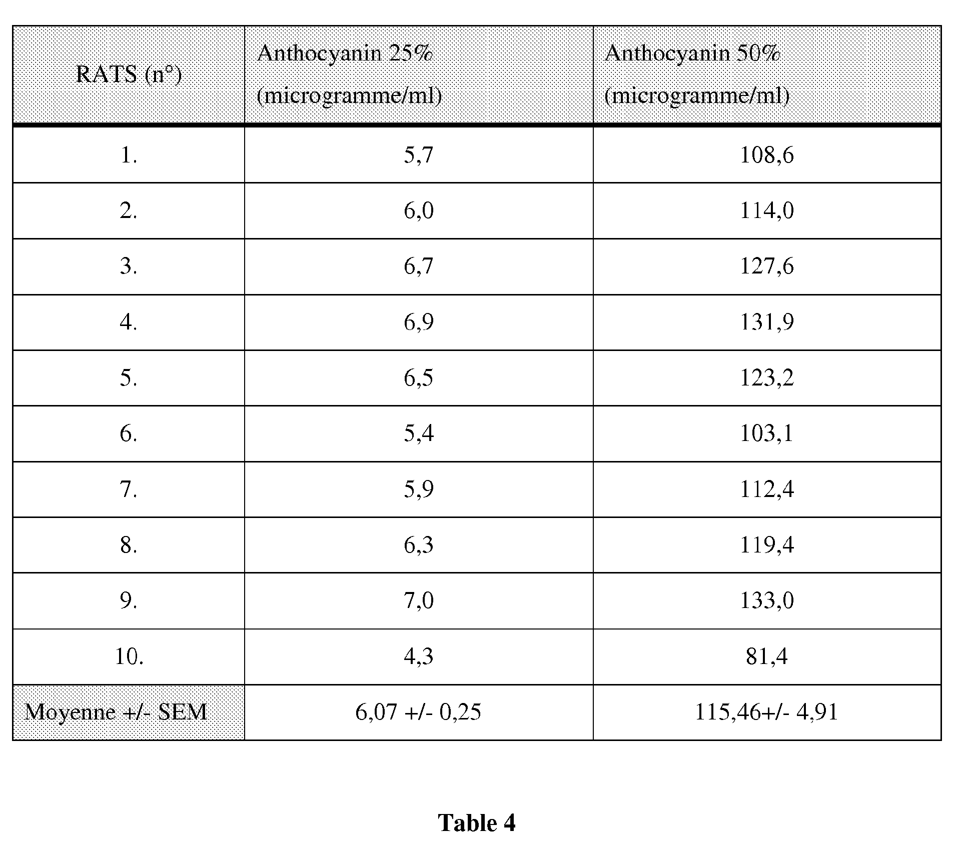

Comparison of buccal and digestive absorption of anthocyanins is presented on figure 1. Anthocyanins are significantly absorbed in the tissues and in the plasma. Moreover, the absorption is more effective when the administration is done through the lingual way, as compared to the digestive way.

## Claims

1. A composition comprising a natural extract containing anthocyanins and an agent for enhancing vigilance, said composition being in a form adapted to transmucosal administration, said form preferably being a chewing gum, an orodispersible/orodisintegrating tablet or a buccal spray, for alleviating a visual discomfort.

2. A composition according to claim **1,** wherein said composition is a nutraceutical composition, a food or dietary supplement or a functional food.

3. A composition according to claim **1** and **2,** wherein said transmucosal administration is a buccal administration.

4. A composition according to anyone of claims **1** to **3,** wherein said agent for enhancing vigilance is at least one of caffeine, taurin and vitamin C, preferably caffeine.

5. A composition according to anyone of claims **1** to **4,** wherein said composition is such that a daily amount ranging from 0.01 to 600 mg of natural extract containing anthocyanins, preferably from 0.1 to 50 mg, more preferably from 0.5 to 20 mg may be administered to the subject, in another embodiment, the composition is such that a daily amount ranging from 0.01 to 600 mg of natural extract containing anthocyanins, preferably from 1 to 160 mg, more preferably from 10 to 100 mg may be administered to the subject.

6. A composition according to anyone of claims **1** to **5,** wherein said natural extract containing anthocyanins is a vegetal extract, said vegetal being selected from the group comprising, but not limited to, *Vaccinium Myrtillus* (bilberries i.e. "bog whortleberries"), *Rubus spp* (blackberry i.e. "boysenberries" from north America), *Rubus occidentalis* (black raspberry from north America), *Vaccinium corymbosum* (blueberry), *Vaccinium macrocarpon* (American cranberry), *Vaccinium oxycoccus* (European cranberry), *Rubus idaeus* (European cranberry), *Fragaria X ananassa* (European cranberry), *Prunus virginiana* (berry i.e. "chokecherry" from North American tribal communities), *Viburnum trilobum* (berry i.e. "highbush cranberry " from North American tribal communities), *Amelanchier alnifolia* (berry i.e. "serviceberry " from North American tribal communities), *Shepherdia argentea* (berry i.e. "silver buffaloberry " from North American tribal communities), *Rubus articus* (arctic bramble), *Ribes nigrum* (black currant), *Rubus chamaemorus* (cloudberries), *Empetrum nigrum, Empetrum hermaphroditum* (crowberries), *Sambucus spp.* (elderberries), *Ribes uva-crispa* (gooseberry), *Vaccinium vitis-idaea* (lingonberries), *Rubus loganobaccus* (loganberry), *Sorbus spp* (Rowan berries), *Hippophae rhamnoides* (sea buckthorn), *Punica granatum* (pomegranate), *Lycium barbarum* (goji berries), *Garcinia mangostana* (mangosteen), *Euterpe oleraceae* (Brazilian açaí berry), *Aristotelia chilensis* (Chilean maqui berry).

7. A composition according to anyone of claims **1** to **6,** wherein natural extract containing anthocyanins is a bilberry extract, preferably said bilberry belongs to the Ericaceae family, gender *Vaccinium,* more preferably the bilberry is *Vaccinium myrtillus.*

8. A composition according to anyone of claims **1** to **7,** wherein natural extract containing anthocyanins is a gooseberry extract, preferably said gooseberry belongs to the Grossulariaceae family, gender *Ribes.*

9. A composition according to anyone of claims **1** to **8,** wherein said composition is such that a daily amount ranging from 0.1 to 200 mg of caffeine, preferably from 1 to 150 mg, more preferably from 10 to 100 mg may be administered to the subject.

10. A composition according to anyone of claims **1** to **9,** further comprising one or more of the following ingredients:
- Vitamin C, in an amount ranging from 0.1 to 1000 mg, preferably from 1 to 300 mg, more preferably from 10 to 280 mg, even more preferably about 240 mg; according to another embodiment, the amount of Vitamin C ranges from 0.1 to 240 mg,
- lutein and zeaxanthin in a 5/1 ratio, in an amount ranging from 0.01 to 12 mg, preferably from 0.05 to 6 mg, more preferably from 0.1 to 1.2 mg,
- taurin, in an amount ranging from 0.1 to 200 mg, preferably from 1 to 100 mg, more preferably from 10 to 50 mg,
the indicated amounts being the daily amount to be administered.

11. A composition according to anyone of claims **1** to **10,** wherein lutein and/or zeaxanthin are partially or totally substituted with meso-zeaxanthin, astaxanthin or a mix thereof.

12. A composition according to anyone of claims **1** to **11,** wherein visual discomfort is related to oxidative stress, said oxidative stress being due for example to photo-oxidation or photoreceptor overstimulation, especially of the retina.

13. A composition according to anyone of claims **1** to **12,** wherein said visual discomfort is a poor night vision, an enhanced light sensitivity or glare.

14. A method for alleviating visual discomfort related to or caused by oxidative stress comprising administering the composition according to anyone of claims **1** to **13.**

15. Chewing gum comprising a natural extract containing anthocyanins and an agent for enhancing vigilance, for alleviating a visual discomfort, comprising :
- 0.1 to 50 mg of natural extract containing anthocyanins, preferably 1-5,
- 1 to 150 mg of caffeine, preferably 5 to 20 mg,
- 0 to 200 mg of Vitamin C,
- 0.01 to 1 mg of lutein and/or zeaxanthin, preferably 0.05 to 2 mg,
- 0 to 200 mg of taurin.
